# EUROPEAN PATENT APPLICATION

(11) **EP 4 023 164 A1**
(43) Date of publication of application: **06.07.2022**
(21) Application number: 20858231.2
(22) Date of filing: 07.08.2020
(51) Int. Cl.: A61B 17/04, A61F 2/24

(54) **LOCKING NAIL FOR LOCKING SUTURE AND INTERVENTIONAL REMOTE SUTURE LOCKING DEVICE**

(30) Priority: 29.08.2019 CN 201910811137
(71) Applicant: Hangzhou Valgen Medtech Co., Ltd., Hangzhou, Zhejiang 310052 (CN)
(72) Inventor: ZHANG, Tingchao, Hangzhou, Zhejiang 310052 (CN); ZHANG, Weiwei, Hangzhou, Zhejiang 310052 (CN); ZHENG, Xianzhang, Hangzhou, Zhejiang 310052 (CN); LI, Liguang, Hangzhou, Zhejiang 310052 (CN)
(74) Representative: Hamer, Christopher K.
(86) International application number: PCT/CN2020/107994
(87) International publication number: WO 2021/036761

(57) **Abstract**

A locking nail (300) for locking sutures (500) includes a locking portion (310). The locking nail (300) has a threading cavity (301) used to allow the sutures (500) to extend therethrough. The threading cavity (301) penetrates through the locking portion (310). The threading cavity (301) has a first inner cavity section (302) located at the locking portion (310). A surface roughness of an inner circumference surface of the first inner cavity section (302) ranges from 0.1 µm to 2.5 µm. The surface roughness of the inner circumference surface of the locking portion (310) is controlled, so that a locking force of the suture (500) locked in the first inner cavity section (302) of the locking nail (300) is suitable, thereby not only meeting requirements for the locking force but also preventing the sutures (500) from being damaged and broken due to excessive squeezing. The friction between the sutures (500) and the inner circumference surface of a second inner cavity section (304) is not easy to cause wear of the sutures (500), thereby preventing the sutures (500) from being damaged. This application further provides an interventional remote suture locking device (100) using the locking nail (300).

## Description

### TECHNICAL FIELD

The disclosure relates to the technical field of medical instruments, in particular to a locking nail for locking sutures, and an interventional remote suture locking device using the locking nail.

### BACKGROUND

A mitral valve is a one-way valve between the left atrium (LA for short) and the left ventricle (LV for short) of the heart. The normal and healthy mitral valve can control blood to flow from LA to LV, while preventing the blood flowing from LV to LA. The mitral valve has a pair of leaflets, called the anterior leaflet and the posterior leaflet. When LA is in a diastole state, the anterior leaflet and the posterior leaflet are opened, the blood flows from LA to LV. When LV is in a systole state, edges of the anterior leaflet and the posterior leaflet are closed, the mitral valve may be fully closed to prevent the blood flowing from LV to LA. When the mitral valve leaflets and associated structures thereof have organic or functional lesions, insufficient coaptation of the anterior leaflet and the posterior leaflet of the mitral valve may be caused. Thus, during systole of LV, the mitral valve cannot close sufficiently. As a result, the blood flows back from LV to LA, causing a series of pathological and physiological changes, which are referred to as "mitral valve regurgitation".

In a related art, one or a plurality of sutures can be implanted into the anterior leaflet and/or posterior leaflet of the mitral valve respectively, and tail ends of the sutures can be fixed on the ventricular wall, heart apex, or papillary muscle. The sutures serve as the chordae tendineae to achieve chordae tendineae repair, or the plurality of sutures on the two leaflets are fixed together to achieve "edge-to-edge" repair. Commonly used sutures are made of polytetrafluoroethylene (PTFE for short), expanded polytetrafluoroethylene (e-PTFE for short), polyethylene terephthalate (PET for short), polyethylene (PE for short), polypropylene (PP for short), or the like. These sutures are applicable to various types of soft tissue closure and ligation, such as cardiovascular surgery, dentistry, general surgery, and dural repair. Since fixation of the sutures are usually achieved by manually knotting by doctors, tightness of each suture is not easy to control during the manual knotting, which affects surgical results.

At present, a suture locking device that uses a locking nail to lock the suture is also provided. The suture locking device uses a locking nail with a hollow inner cavity, the locking nail is allowed to deform by compressing so as to fix the suture threading therein. In a mitral valve repair surgery, after an operator implants the sutures in the anterior leaflet and/or posterior leaflet of the mitral valve, the sutures can be fixed to the portions such as the ventricular wall, the papillary muscle, or the heart apex with the locking nail to achieve "chordae tendineae repair", or multiple sets of sutures can be fixed together by means of the locking nail to achieve "edge-to-edge" repair. However, after the sutures are fixed through the locking nail, since the heart is always performing a strong contraction motion, the sutures and the locking nail are also pulled accordingly. If a locking force between the locking nail and the sutures is insufficient or the locking force is excessive, breakage and loosening of the sutures or slipping of the locking nail may be caused, which not only affects the surgical results but even endangers the lives of patients in severe cases.

### SUMMARY

The technical problem to be solved by the disclosure is to provide a locking nail for locking sutures for overcoming defects of the related art. By controlling a surface roughness of an inner cavity of the locking nail, the locking nail achieves a suitable locking force, which can lock the sutures and can also avoid damaging the sutures. The disclosure further provides an interventional remote suture locking device.

In order to solve the above technical problem, the disclosure first provides a locking nail for locking sutures. The locking nail includes a locking portion. The locking nail has a threading cavity used to allow the sutures to extend therethrough. The threading cavity penetrates through the locking portion. The threading cavity has a first inner cavity section located at the locking portion. A surface roughness of an inner circumference surface of the first inner cavity section ranges from 0.1 µm to 2.5 µm.

The disclosure further provides an interventional remote suture locking device. The interventional remote suture locking device includes a locking nail and a collet component. The locking nail includes a locking portion. The locking nail has a threading cavity used to allow the sutures to extend therethrough. The threading cavity penetrates through the locking portion. The threading cavity has a first inner cavity section located at the locking portion. A surface roughness of an inner circumference surface of the first inner cavity section ranges from 0.1 µm to 2.5 µm. The locking nail is inserted in the collet component. The collet component is used for compressing the locking nail to cause the locking portion to deform, so as to lock the sutures inserted in the threading cavity of the locking nail.

The interventional remote suture locking device provided by this disclosure adopts the locking nail including the locking portion. The surface roughness of the inner circumference surface of the first inner cavity section ranges from 0.1 µm to 2.5 µm. Accordingly, a locking force to the sutures locked in the first inner cavity section of the locking nail is suitable, thereby not only meeting requirements for the locking force but also preventing the sutures from being damaged and broken due to excessive squeezing.

### BRIEF DESCRIPTION OF THE DRAWINGS

In order to describe the technical solutions in this application more clearly, the accompanying drawings required to be used in the implementations will be simply introduced below. It is apparent that the accompanying drawings in the following descriptions are only some implementations of this application. Those of ordinary skill in the art may further obtain other apparent variations according to these accompanying drawings without creative work.
Fig. 1 is a schematic perspective structural view of an interventional remote suture locking device according to an implementation of the disclosure.
Fig. 2 is a cross-sectional view of Fig. 1 taken along line II-II.
Fig. 3 is a schematic perspective structural view of a locking nail used in the interventional remote suture locking device in Fig. 1.
Fig. 4 is a cross-sectional view of Fig. 3 taken along line IV-IV.
Fig. 5 is a cross-sectional view of Fig 3 taken along line V-V.
Fig. 6 is an enlarged view of part VI in Fig. 2.
Fig. 7 is a schematic structural view of the locking nail in Fig. 3 when sutures are inserted.
Fig. 8 is a schematic view showing a state in which the locking nail in Fig. 7 is deformed to lock the sutures.
Fig. 9 is a cross-sectional view of Fig. 8 along line IX-IX.
Figs. 10-12 are schematic diagrams showing an edge-to-edge repair process for the diseased mitral valve, which is carried out by an interventional remote suture locking device according to an implementation of the disclosure.
Figs. 13-14 are schematic diagrams showing a process of fixing the sutures in the locking nail, which is carried out by the interventional remote suture locking device according to an implementation of the disclosure.
Fig. 15 is an enlarged view of part XV in Fig. 12.
Fig. 16 is a statistical chart of results of a surface roughness test, a deformation rate test, a locking force test, and a fatigue test of the locking nail in the disclosure and locking nails in comparative examples.

### DETAILED DESCRIPTION

The technical solutions in the implementations of this application are clearly and completely described in the following in conjunction with the accompanying drawings of this application. It is apparent that the described implementations are only part of the implementations of this application, not all of the implementations. On the basis of the implementations of this application, all other implementations obtained on the premise of no creative work of those of ordinary skill in the art shall fall within the scope of protection of this application.

In addition, the following explanation of each implementation refers to illustration of an implementable specific implementation of this application with reference to additional drawings. The direction terms mentioned in this application, such as "up", "down", "front", "back", "left", "right", "inner", "outer", and "side" are only the directions with reference to the additional drawings. Therefore, the used direction terms are intended to better and more clearly illustrate and understand this application instead of indicating or implying that the device or element must have a specific orientation or must be constructed and operated in a specific orientation, and thus cannot be interpreted as limitation to this application. In addition, "axial direction" refers to a direction of an axis of a pusher or a direction of an axis of a push tube.

Definition of orientations: for clarity of description, hereinafter, during an operation, one end close to an operator is referred to as a "proximal end", one end away from the operator is referred to as a "distal end", and "axial direction" indicates a direction parallel to a line between a distal center and a proximal center of a medical instrument. The above definitions are only for the convenience of presentation and should not be understood as limits to this application.

The implementation takes an occluder for blocking the aortic dissection tear as an example of an interventional medical instrument to illustrate a mechanism and application of an interventional medical instrument pushing device of this application. However, the application range of the interventional medical instrument pushing device of this application is not limited to the occluder.

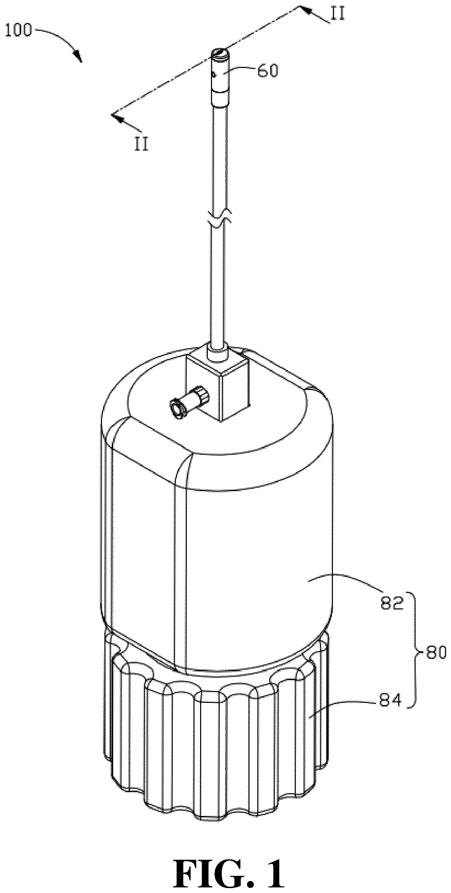

Referring to Fig. 1 to Fig. 6, the disclosure provides an interventional remote suture locking device 100 for fixation between sutures and a locking nail 300. The interventional remote suture locking device 100 includes a collet component 20, a driving component 40 arranged at a proximal end of the collet component 20, and a pushing component 60 sleeved outside the collet component 20 and the driving component 40. A gap 25 for holding the locking nail 300 is formed at a distal end of the collet component 20. The locking nail 300 defines a threading cavity 301 which axially extends therethrough. The interventional remote suture locking device 100 defines a threading passage 26 which axially extends therethrough and is used for the collet component 20 and the driving component 40 to pass through. The locking nail 300 includes a locking portion 310 and a retaining portion 330 arranged at a distal end of the locking portion 310. The threading cavity 301 penetrates through the locking portion 310 and the retaining portion 330. The locking portion 310 is accommodated in the gap 25 of the collet component 20. The retaining portion 330 is retained on a distal surface of the pushing component 60. The threading cavity 301 includes a first inner cavity section 302 located in the locking portion 310, and a second inner cavity section 304 located in the retaining portion 330. A surface roughness of an inner circumference surface of the first inner cavity section 302 is greater than or equal to that of an inner circumference surface of the second inner cavity section 304. The sutures are used to pass through the threading cavity 301 and the threading passage 26 of the locking nail 300. The driving component 40 is rotated relative to the pushing component 60 and axially moved to push the collet component 20 to compress the locking nail 300 placed in the gap 25, so that the locking nail 300 is deformed to fix the sutures. Since the surface roughness of the inner circumference surface of the first inner cavity section 302 of the locking nail 300 is greater than that of the inner circumference surface of the second inner cavity section 304, the sutures in the first inner cavity section 302 of the locking nail 300 are subjected to a high locking force. The sutures are not easy to wear by friction between the sutures and the inner circumference surface of the second inner cavity section 304, thereby avoiding being damaged.

The interventional remote suture locking device 100 further includes a handle 80 connected to a proximal end of the driving component 40 and a proximal end of the pushing component 60. The handle 80 includes a fixed portion 82 at a distal end of the interventional remote suture locking device 100 and a movable portion 84 at a proximal end of the interventional remote suture locking device 100. The fixed portion 82 is capable of moving relative to the fixed portion 82. The fixed portion 82 is fixedly connected to the proximal end of the pushing component 60. The movable portion 84 is connected to the proximal end of the driving component 40. The movable portion 84 and the fixed portion 82 are in relative movement to drive the driving component 40 to axially move relative to the pushing component 60. In an implementation, the movable portion 84 and the fixed portion 82 can relatively rotate and move in an axial direction to drive the driving component 40 to rotate relative to the pushing component 60 and move in the axial direction. When the driving component 40 is axially moved toward the distal end to push the collet component 20, the locking nail 300 placed in the gap 25 may be compressed, so that fixation between the sutures and the locking nail 300 is completed.

The locking nail 300 is made of a biocompatible material such as stainless steel, pure titanium, nickel titanium, or cobalt-chromium alloy. Preferably, the locking nail 300 is made of pure titanium or stainless steel.

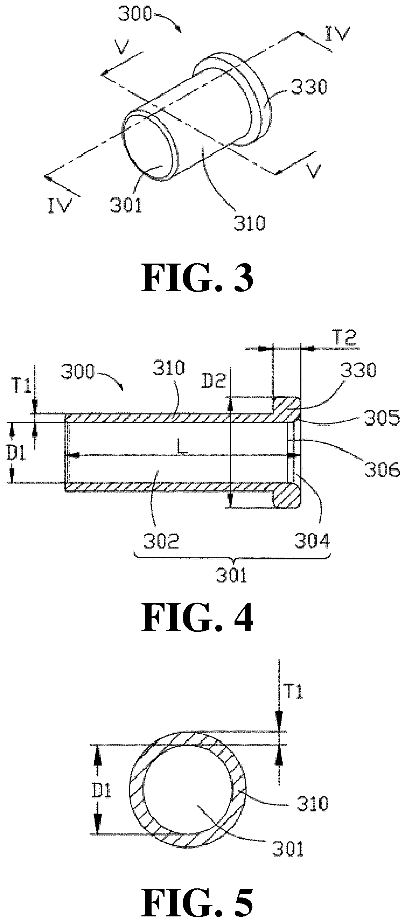

Referring to Fig. 3 to Fig. 5, the threading cavity 301 of the locking nail 300 axially penetrates two opposite ends of the locking nail 300, and is used for receiving and threading of the sutures. The locking portion 310 may be dented under action of an external mechanical force so as to fix the sutures in the threading cavity 301 of the locking nail 300. The cross section of the threading cavity 301 of the locking portion 310 may be in various shapes, for example, cylindrical, prismatic, elliptical, polygonal, or irregular shape, as long as the threading cavity 301 is provided and used for receiving the sutures. A fillet is formed at an edge of a proximal surface of the locking portion 310, which is smooth in surface, that is, is of a small roughness, so that the proximal surface of the locking portion 310 is prevented from damaging the tissue.

In other implementations, the first inner cavity section 302 has a lock boss that is protrusive and a lock groove that is recessed at opposite positions of the inner circumference surface of the first inner cavity section 302. The locking portion 310 is capable of being deformed when the locking nail 300 is subjected to an external force, so as to allow the lock boss to be pressed into the lock groove. When the locking nail 300 continues to deform, the lock boss and the lock groove are simultaneously deformed until they cannot be separated. Here, sutures 500 are firmly fixed in the threading cavity 301 of the locking nail 300.

The first inner cavity section 302 serves a main area of the locking nail 300 for locking the sutures. An inner surface of the first inner cavity section 302 of the locking nail 300 is in direct contact with the sutures, so that the surface roughness of the inner circumference surface of the first inner cavity section 302 directly affects the locking force of the locking nail. The surface roughness refers to unevenness with small spacings and small peaks and valleys on a processed surface of a material. The smaller the surface roughness, the smoother the surface of the material. The surface roughness is generally formed by processing methods, surface treatments, and other factors used, such as s friction between tools and surfaces of parts during processing, plastic deformation of metal on surface coats during chip separation, and highfrequency vibration in processing systems. The surface treatment methods include physical polishing, chemical polishing, electroplating, sandblasting, spraying, electrical discharge machining, and the like. Due to the difference between the processing methods and the surface treatment methods, depths, densities, shapes, and textures of traces left on the processed surfaces are different. The surface roughness of the inner surface of the first inner cavity section 302 mainly affects the locking nail 300 in the following aspects.

Firstly, the locking force of the locking nail 300 is affected. In an implementation, the effect of the surface roughness to the locking force mainly lies in affecting matching stability of the first inner cavity section 302 of the locking nail 300. When the sutures are inserted in the locking nail 300 and the locking nail 300 is deformed under pressing, inner cavities of the locking portion 310 of the locking nail 300 are in clearance fit, the rougher the surfaces of the inner cavities, the easier they are to wear. When the sutures are locked, along with constant motions of the human tissue, the clearance is gradually increased, and a connection strength is gradually reduced. The inner circumference surface of the locking portion 310 of the locking nail 300 is in interference fit with the sutures. Microcosmic peaks of the locking portion 310 are flattened by compressing, so that the actual effective interference is reduced, and the connection strength is decreased.

Secondly, a fatigue strength of the locking nail 300 is affected. Because a rough surface always has big valleys, which is very sensitive to stress concentration, for a surface with a large roughness, an effective contact area between matching surfaces is small, resulting a large pressure, a large friction resistance, rapid wear, and a poor wear resistance. Moreover, the rougher surface is likely to cause the blood to permeate the inner layer of the locking nail 300 through microscopic valleys on the surface, resulting in surface corrosion.

Thus, the surface roughness of the inner circumference surface of the first inner cavity section 302 may be designed according to the locking force needed for the sutures 500. In order to ensure a balance between the locking force and the fatigue strength as well as safety and effectiveness of the locking nail 300, the surface roughness of the inner circumference surface of the first inner cavity section 302 ranges from 0.1 µm to 2.5 µm.

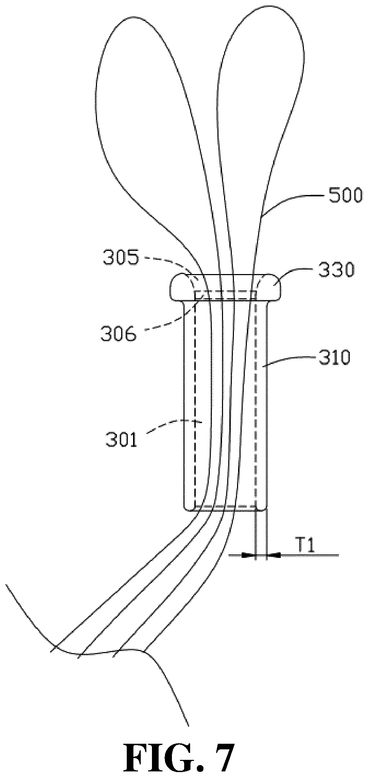

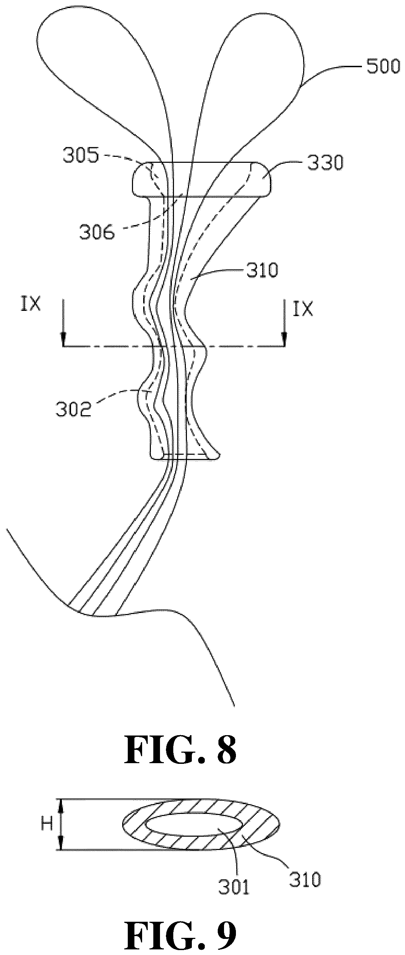

Referring to Fig. 7 to Fig. 9, after the sutures 500 are inserted in the threading cavity 301 of the locking nail 300, the collet component 20 compresses the locking nail 300 so that the locking nail 300 is deformed to fix the sutures 500. Therefore, a deformation rate of the locking nail 300 may affect the locking force and fatigue resistance of the locking nail 300. The deformation rate of the locking nail 300 reflects a deformation degree of the locking nail 300 after being compressed. In the disclosure, after the locking portion 310 of the locking nail 300 is compressed, a thickness of a minor axis of a cross section of the threading cavity 301, used to fix the sutures 500, of the locking portion 310, at the smallest dimension in a direction perpendicular to an axial direction of the threading cavity 301 is denoted by H, a unilateral wall thickness of the locking nail 300 in an initial state is denoted by T1, and thus the deformation rate of the locking nail 300 is expressed as ε=2W/H × 100%. The deformation rate ε may reflect a size of a internal gap of the threading cavity 301 after the locking nail 300 is deformed by compressing. If the deformation rate of the locking nail 300 is larger, then the H valve is smaller, which indicates that the gap is smaller after the locking nail 300 is deformed, and the locking force is higher. However, if the deformation rate is too large, it indicates that the gap is too small after the locking nail 300 is deformed, which may cause breakage of the sutures 500, or affect the fatigue strength of the locking nail 300. In the disclosure, the deformation rate of the locking portion 310 ranges from 50% to 90%, so as to ensure the locking force of the locking nail 300.

An e-PTFE suture is a commonly used surgical suture, which is chemically synthesized from 100% polytetrafluoroethylene, is a single-strand non-absorbable surgical suture with features such as high porosity, high smoothness, and low friction coefficient. In a manual knotting process of the e-PTFE suture, the suture is tightened usually by passing more than two loops. When the suture is fixed by a metal locking nail, the locking force is required to be appropriate to avoid slippage caused by the insufficient locking force. Since the e-PTFE suture is a single-strand thread with poor wear resistance correspondingly, it is necessary to avoid excessive locking force breaking the suture, and to ensure the fatigue resistance of the locking nail in cases that the heart continues to beat and the suture and the locking nail are repeatedly pulled after the locking nail is combined with the e-PTFE suture. In the implementation, the deformation rate preferably ranges from 55% to 80%. By matching the surface roughness of the inner circumference surface of the locking portion 310 with the deformation rate of the locking portion 310, a balance between he locking force and the fatigue resistance is achieved, which is especially suitable for locking and fixation of a single-strand suture such as the e-PTFE suture. In other implementations, the deformation rate further preferably ranges from 60% to 75%, which is especially suitable for locking of 2 to 6 e-PTFE sutures.

A retaining portion 330 is arranged at the distal end of the locking portion 310. In an implementation, at least part of the retaining portion 330 is radially protruded from an outer wall of the distal end of the locking portion 310. That is, an outside diameter of the retaining portion 330 is greater than that of the locking portion 310. That is, the cross-sectional area of the distal surface of the retaining portion 330 is larger than the radial cross-sectional area of the locking portion 310. Therefore, the retaining portion 330 has a position limiting function to prevent a distal surface of the locking pin 300 from being pulled into or sliding into the gap 25 of the collet component 20 when the distal surface of the locking pin 300 is compressed and deformed. In the implementation, the retaining portion 330 is a boss with an annular cross section. In the related art, the distal end of the locking pin 300 is provided with no retaining portion 330. When the distal surface of the locking pin 300 is compressed and deformed, it is easy to be pulled or to slide into the gap 25 of the collet component 20. When the collet component 20 is unfolded to return to the initial position, the distal surface of the locking pin 300 may be caught by the collet component 20, and the locking pin 300 cannot be automatically separated from the distal end of the interventional remote suture locking device 100.

The retaining portion 330 defines a second inner cavity section 304 therein which is in communication with the first inner cavity section 302. That is, the second inner cavity section 304 and the first inner cavity section 301 constitute a threading cavity for threading sutures. A surface roughness of the inner circumferential surface of the second inner cavity section 304 is less than or equal to that of the inner circumferential surface of the first inner cavity section 302, thereby reducing a friction between the sutures and the inner circumferential surface of the second inner cavity section 304, to avoid long-term friction damage to the sutures.

Both peripheral edges of the distal end and the proximal end of the retaining portion 330 are provided with fillets. The outer surface of the retaining portion 330 is a curved surface, thereby preventing the locking nail 300 from scratching the internal tissue of the patient's body. In the implementation, the retaining portion 330 is a boss with an annular cross section, and the outer surface of the boss is a curved surface. A fillet is arranged at an intersection of the distal surface of the boss and the inner circumferential surface of the second inner cavity section 304.

A first smooth transition area 305 is arranged at an intersection of the distal surface of the retaining portion 330 and the inner circumference surface of the first inner cavity section 302. The sutures locked in the threading cavity 301 of the locking nail 300 are in contact with the first smooth transition area 305 at a certain inclined angle, so that the sutures are prevented from being cut at the intersection of the distal surface of the retaining portion 330 and the inner circumference surface of the first inner cavity section 302, and prevented from friction fatigue or even breakage. In the implementation, the first smooth transition area 305 is processed by electrical discharge machining, and its surface roughness ranges from 0.1 µm to 2.5 µm to avoid damage to the sutures. In other implementations, the first smooth transition area 305 may also be subjected to a coating process after electrical discharge machining, to further reduce the roughness. By PTFE film coating, the surface roughness of the first transition area 305 ranges from 0.1 µm to 0.5 µm.

As shown in Fig. 4, a second smooth transition area 306 is arranged between the first smooth transition area 305 and the inner circumference surface of the first inner cavity section 302. A surface roughness of the second smooth transition area 306 is less than 2 µm. If the surface roughness of the second smooth transition area 306 is too large, the sutures may be subjected to friction fatigue and even be broken. The surface roughness of the second smooth transition area 306 is controlled within 2 µm, which can effectively avoid breakage of the sutures.

In order to reduce the roughness of the second smooth transition area 306, the second smooth transition area 306 may be subjected to the electrical discharge machining. In order to further reduce the roughness of the second smooth transition area 306, a PTFE coating process may be added on the basis of the electrical discharge machining.

In order to further reduce the roughness or offset of the second smooth transition area 306, the second smooth transition area 306 is a conical surface with an inside diameter gradually decreasing from the distal end to the proximal end, and a unilateral slope of the conical surface ranges from 0.3 degree to 0.8 degree, and preferably ranges from 0.5 degrees to 0.6 degrees.

Referring to Fig. 4 and Fig. 5, an inside diameter D1 of the threading cavity 301 of the locking nail 300 is determined according to the thread diameter and number of sutures to be locked. The inside diameter D1 of the threading cavity 301 ranges from 0.8 µm to 1.2 µm. currently, an outside diameter of an e-PTFE suture is commonly 0.3 µm, and the number of the e-PTFE sutures to be locked is commonly 2 to 4. Preferably, the inside diameter D1 of the threading cavity 301 ranges from 0.85 µm to 1.0 µm. A wall thickness T1 of the locking portion 310 of the locking nail 300 ranges from 0.1 µm to 0.2 µm. If the wall thickness T1 of the locking portion 310 is less than 0.1 µm, it is not conducive to the fatigue resistance of the locking nail 300. If the wall thickness T1 of the locking portion 310 is greater than 0.2, it is not conductive to deformation and locking strength of the locking nail 300. Preferably, the wall thickness T1 of the locking portion 310 ranges from 0.12 µm to 0.18 µm.

A thickness T2 of the boss of the retaining portion 330 ranges from 0.3 µm to 0.6 µm. Preferably, the thickness T2 of the boss of the retaining portion 330 ranges from 0.4 µm to 0.5 µm. An outside diameter D2 of the boss of the retaining portion 330 ranges from 1.6 µm to 2.2 µm. Preferably, the outside diameter D2 of the boss of the retaining portion 330 ranges from 1.8 µm to 2.0 µm. An overall length L of the locking nail 300 ranges from 4 µm to 6.5 µm. If the overall length L of the locking nail 300 is too long, it is not conducive to delivery, and may also affect the safety after the locking nail 300 is implanted in the human body. Preferably, the overall length L of the locking nail 300 ranges from 4.5 µm to 6.0 µm. The overall length L of the locking nail 300 minus the thickness T2 of the boss of the retaining portion 330 to obtain the length of the first inner cavity section 302.

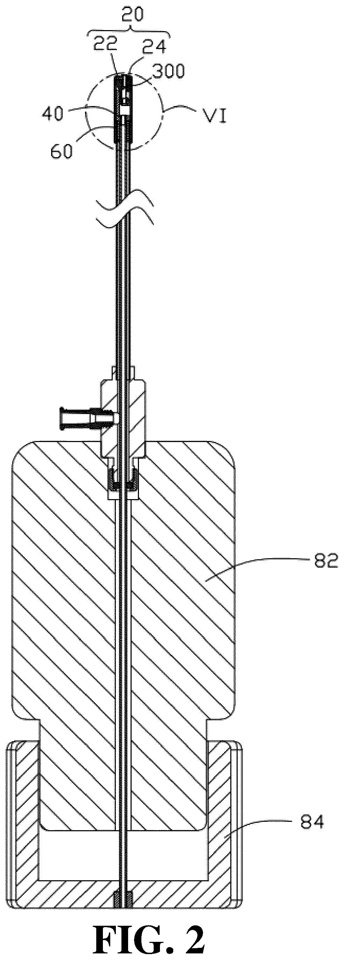

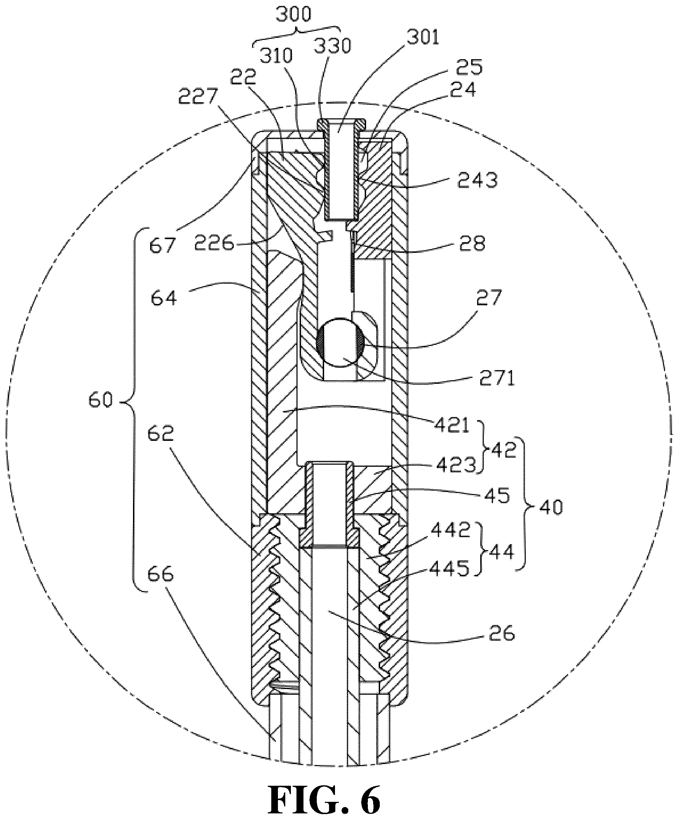

Referring to Fig. 2 and Fig. 6, the collet component 20 includes a first collet 22 and a second collet 24 that are connected to each other or integrally molded, and a joint between the first collet 22 and the second collet 24 is in communication with the threading passage 26. The gap 25 is defined between a distal end of the first collet 22 and a distal end of the second collet 24. The locking pin 300 is placed in the gap 25, that is, the locking pin 300 is inserted in the collet component 20. When the driving component 40 rotates relative to the pushing component 60 and moves in the axial direction to push the first collet 22 and the second collet 24 of the collet component 20 to rotate toward each other, the first collet 22 and the second collet 24 can squeeze the locking nail 300, so that the locking nail 300 is deformed to lock the sutures in the threading cavity 301 of the locking nail 300.

In the implementation, a proximal end of the first collet 22 and a proximal end of the second collet 24 are rotatably connected through a rotating shaft 27. The rotating shaft 27 defines a through hole 271 therein which is in communication with the threading passage 26. An inclined slide guiding surface 226 is arranged on one side, away from the second collet 24, of the first collet 22, and is located at the distal end of the first collet 22 and extends to one side away from the threading passage 26. A first clamp tooth 227 is arranged at a position, near the distal end, on a lateral side, facing the second collet 24, of the first collet 22. The first clamp tooth 227 includes a plurality of tooth grooves, and each tooth groove extends in the axial direction substantially parallel to a shaft hole 224. A second clamp tooth 243 is arranged at a position, near the distal end, on a lateral side, facing the first collet 22, of the second collet 24. The second clamping tooth 243 includes a plurality of tooth grooves. An extending direction of each tooth groove of the second clamping tooth 243 is the same as an extending direction of each tooth groove of the first clamp tooth 227. After the first collet 22 and the second collet 24 are rotationally connected by the rotating shaft 27, the first clamp teeth 227 of the first collet 22 and the second clamp teeth 243 of the second collet 24 are misaligned and engaged with each other. Therefore, the first collet 22 rotates toward the second collet 24, and the first clamp teeth 227 and the second clamp teeth 243 squeeze the locking nail 300 placed in the gap 25 into a shape having a curvature. The collet component 20 further includes a resilient element 28. The resilient element 28 is used for rotation resetting of the first collet 22 and/or the second collet 24, thereby facilitating inserting the locking nail 300 into the gap 25 between the first collet 22 and the second collet 24, and resetting the first collect 22 after the locking nail 300 is squeezed by the first collect 22 and the second collet 24 so as to smoothly release the locking nail 300.

A driving member 44 includes a screw 442 and a rotating mandrel 445 axially connected to the screw 442. In an implementation, a distal end of the rotating mandrel 445 is fixedly connected to the screw 442, and a proximal end of the rotating mandrel 445 is fixedly connected to the movable portion 84. A rotation of the movable portion 84 can drive the rotating mandrel 445 and the screw 442 to rotate together.

An ejector member 42 includes an ejector rod 421 for being slidingly abutted against the slide guiding surface 226 of the first collet 22 in the axial direction, and a connecting block 423 arranged at a proximal end of the ejector rod 421. The ejector rod 421 is arranged on one side of the connecting block 423 in the radial direction. The connecting block 423 has a through hole along the axial direction, the through hole penetrates through a distal surface and a proximal surface of the connecting block 423. A connecting pin 45 is rotatably inserted into the through hole. The pushing component 60 includes a thrust tube 62 rotatably sleeving the screw 442, a front-end outer tube 64 connected to a distal end of the thrust tube 62, a pushing shaft 66 connected to the distal end of the thrust tube 62, and an end cap 67 covering a distal end of the front-end outer tube 64. The thrust tube 62 is provided with an internal thread 622 corresponding to the screw 442. A rotation of the rotating mandrel 445 can drive the screw 442 to rotate relative to the thrust tube 62 and move in the axial direction. Preferably, an internal thread 622 corresponding to the screw 442 is arranged on an inner wall of the thrust tube 62. The distal end of the pushing shaft 66 is fixedly connected to the proximal end of the thrust tube 62. The proximal end of the pushing shaft 66 is fixedly connected to the fixed portion 82.

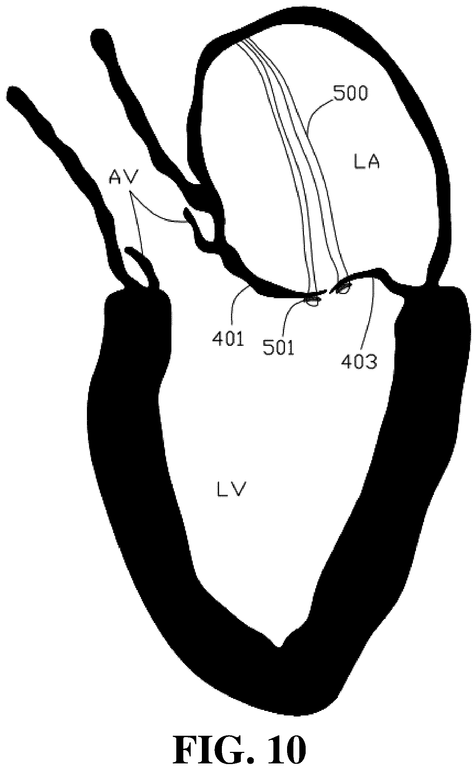

Referring to Fig. 10 to Fig. 15, the following takes a heart mitral valve repair operation as an example to illustrate applications of the interventional remote suture locking device 100 and the locking nail 300, provided by the disclosure, in the mitral valve edge-to-edge repair operation.

At step 1, as shown in Fig. 10, femoral venous puncture is first performed on a patient. After interatrial septal puncture, a plurality of sutures 500 with elastic pads 501 are respectively implanted in the anterior leaflet 401 and the posterior leaflet 403 of the mitral valve. A point contact between the suture 500 and the valve leaflets is converted to a surface contact between the elastic pads 501 and the valve leaflets, so that the risk of leaflet tearing may be reduced effectively.

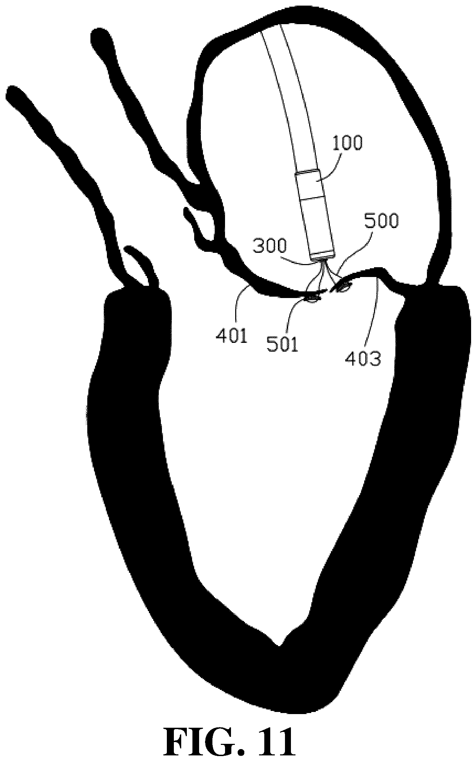

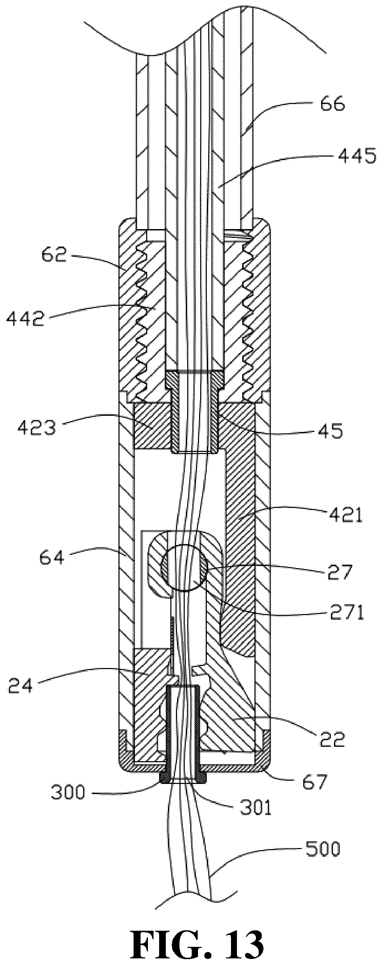

At step 2, as shown in Fig. 11 and Fig. 13, the plurality of sutures 500 on both valve leaflets are inserted, outside the patient's body, into the threading cavity 301 of the locking nail 300, and proximal ends of the sutures 500 sequentially pass through the threading cavity 301 of the locking nail 300 of the interventional remote suture locking device 100, the gap between the first collet 22 and the second collet 24, the through hole 271 of the rotating shaft 27, the through hole of the front-end outer tube 64, a leading hole of the connecting pin 45, and an hollow inner hole of the rotating shaft 445, and then pass out of the proximal end of the movable portion 84.

At step 3, the distal end of the interventional remote suture locking device 100 is pushed into the heart through the femoral vein and the interatrial septum by aid of a bendable sheath (not shown) to move toward to the leaflets of the mitral valve while pulling the sutures 500 until the distal end of the interventional remote suture locking device 100 reaches a predetermined position.

At step 4, the tightness of the sutures 500 on the anterior leaflet 401 and the posterior leaf 403 is adjusted respectively, the least state of mitral valve regurgitation is determined through ultrasound. When this state is reached, the adjustment is stopped, and the tightnesses of the two sets of sutures 500 is maintained, that is, a relative distance between the anterior leaflet 401 and the posterior leaflet 403 of the mitral valve is maintained.

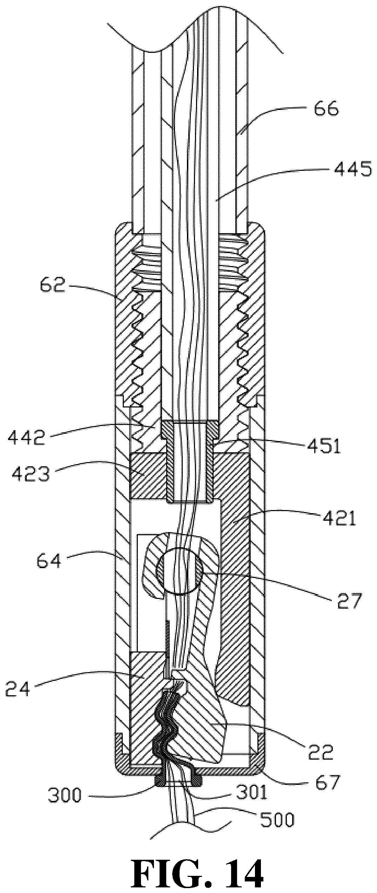

At step 5, as shown in Fig. 11 and Fig. 14, the fixed portion 82 of the handle 80 keeps fixed, and the movable portion 84 is driven to rotate toward the distal end of the interventional remote suture locking device 100. Here, the mandrel 445 is rotated to drive the screw 442 to move toward the distal end of the interventional remote suture locking device 100 relative to the pushing shaft 66. The screw 442 drives the ejector member 42 to move toward the distal end of the interventional remote suture locking device 100, the distal end of the ejector rod 421 of the ejector member 42 continuous to compress the first collet 22, so that the first collet 22 is driven to close to the second collet 24. When the locking nail 300 between the first collet 22 and the second collet 24 is compressed, the resilient element 28 is compressed to elastically deform, until the locking nail 300 is deformed, the locking nail 300 is fixed to the sutures 500 inserted in the threading cavity 301 of the locking nail 300, and the retaining portion 330 is retained outside the end cap 67. After that, the movable portion 84 is driven to move toward the proximal end of the interventional remote suture locking device 100, the compressing to the first collet 22 is released by means of the ejector rod 421, the resilient element 28 of the first collet 22 is expanded under action of resilient resetting and then restores to an initial position, and the deformed locking nail 300 is released from the gap between the first collet 22 and the second collet 24. At this time, the sutures 500 are locked in the first inner cavity section 302 of the locking portion 310 that is deformed, so that the locking force to the sutures 500 is high. In addition, the sutures are in contact with the first smooth transition area 305 and the second smooth transition area 306 of the locking nail 300, and damage to the sutures 500 can be avoided due to the small surface roughness of the first smooth transition area 305 and the second smooth transition area 306.

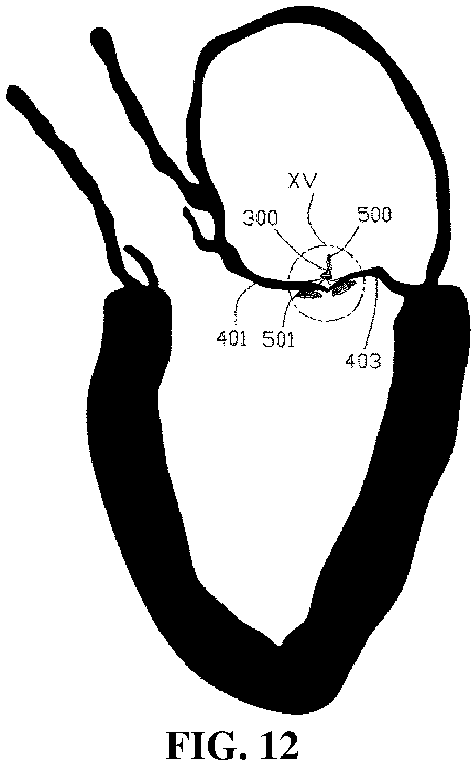

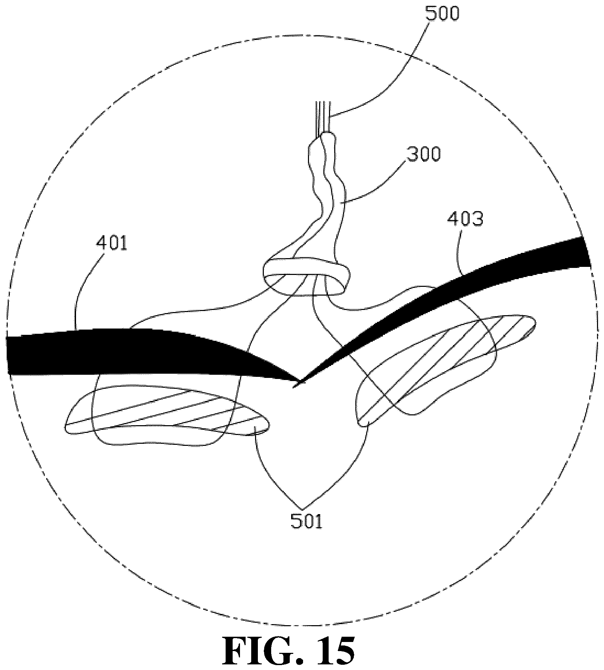

At step 6, as shown in Fig. 12 and Fig. 15, the distal end of the interventional remote suture locking device 100 is withdrawn from the patient's body, the locking nail 300 is remained in the patient's body, and the sutures 500 at a tail end of the locking nail 300 are cut out. Here, the locking nail 300 fixes the two sets of sutures 500 that respectively pierce the anterior leaflet 401 and the posterior leaflet 403 together. The anterior leaflet 401 and the posterior leaflet 403 of the mitral valve form a double-orifice structure. Thus, the edge-to-edge repair is completed.

It can be understood that in the above description, the interventional remote suture locking device 100 which is used for performing the interventional mitral valve repair process through a path of femoral vein-interatrial septum-left atrium-mitral valve is taken as an example to illustrate a use procedure of the disclosure, and the interventional remote suture locking device of the disclosure can also be used for locking and fixing sutures in other surgical procedures.

The interventional remote suture locking device 100 of the disclosure is particularly applicable to the following scenarios.

An interventional mitral valve repair surgery through a path of femoral vein-interatrial septum-left atrium-mitral valve.

An interventional mitral valve repair surgery through a path of femoral artery-aortic arch-aortic valve-left ventricle-mitral valve.

An interventional mitral valve repair surgery through a path of jugular vein-interatrial septum-left atrium-mitral valve.

It is also applicable to the following scenarios: (1) an interventional tricuspid valve repair surgery through a path of femoral vein-right atrium-tricuspid valve; (2) an interventional tricuspid valve repair surgery through a path of jugular vein-right atrium-tricuspid valve. By means of minimally invasive intervention, the interventional remote suture locking device 100 is remotely operated outside the patient's body to fix the sutures 500 implanted on the valve leaflets by the locking nail 300.

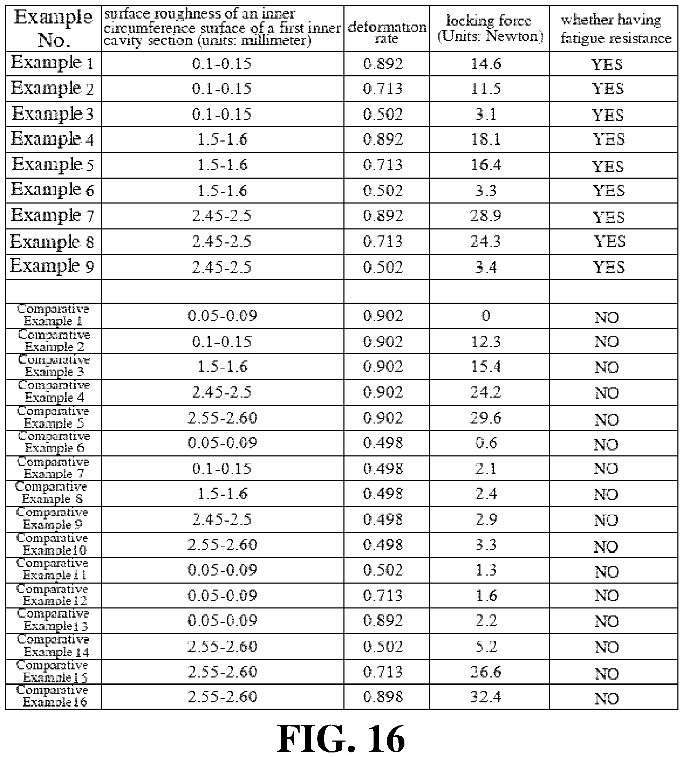

The locking nails in other implementations are of the structures similar to that of the locking nail 300 in Example 1, except that: after preparation of the locking nail, the inner circumference surface of the first inner cavity section of the locking nail is subjected to mirror electric discharge treatment or wire-electrode cutting treatment, so that the surface roughness of the inner circumference surface of the first inner cavity section is different from the surface roughness of the inner circumference surface of the first inner cavity section 302 in Example 1, and the locking nails of Examples 2-9 are obtained respectively, as shown in Fig. 16.

Locking nails with the same size and structure as the locking nail 300 of Example 1 are prepared by the same process, and the surface roughness of the inner circumference surface of each of the locking nails is adjusted to a different range through the mirror electric discharge treatment or wire-electrode cutting treatment. The above identified locking nails serve as locking nails of Comparative Examples 1-16, as shown in Fig. 16.

The locking nails in Examples 1-9 and Comparative Examples 1-16 were subjected to a surface roughness test, a deformation rate test, a locking force test and a fatigue test respectively. Test results are shown in Fig. 16.

### Surface roughness test

Test principle of a stylus method: When a stylus scans gently along a surface to be tested, the stylus moves up and down along peaks and valleys during scanning because of the tiny peaks and valleys on the surface. The movement of the stylus reflects the surface profile.

Test standard: GB/T1031, GB/T10610.

Test instrument: A Dektak 6M probe type surface profiler from Bruker, USA.

Test parameters: probe pressure: 10 mg; scan distance: 800 µm; scan time: 8 seconds, scan speed 100 µm /sec.

Test method: the locking nails 300 of Examples 1-9 and Comparative Examples 1-16 each were cut in half along the axis and divided into two symmetrical parts. 3 to 10 points were respectively taken in the first cavity section 302 of each part. At each sampling point, changes of the surface profile when a probe with a pressure of 10 mg scans 800 µm at a speed of 100 µm/sec were respectively tested. The test results of all sampling points of the two parts were averaged to obtain the surface roughness of the inner circumference surface of the locking nail 300.

### Deformation rate test

Test method: an outside diameter of the locking nail 300 was measured first with a micrometer, an inside diameter of the locking nail 300 was measured with a needle gauge, and then the inside diameter was subtracted from the outside diameter to obtain a bilateral wall thickness, to respectively obtain the bilateral wall thickness 2T1 of the locking nails 300 in an initial state of Examples 1-9 and Comparative Examples 1-16. And then, by the interventional suture locking device 100, the locking nails 300 of Examples 1-9 and Comparative Examples 1-16 were respectively used for fixation of two U-shaped folded e-PTFE sutures 500. After that, a thickness H of a minor axis of the cross section of the locking portion 310, at the smallest dimension in a direction perpendicular to the axial direction of the first inner cavity section 302, of the locking nail 300 after locking was measured by an MS322 optical image measuring instrument manufactured by Shenzhen Zhitai Precision Instrument Co., Ltd., and then the deformation rate of each locking nail was calculated according to ε =2W/H×100%. The results are shown in Fig. 16.

### Locking force test

Test method: The locking nail 300 and the sutures 500 after being locked in the deformation rate test were subjected to the locking force test. The testing equipment is an HY-0580 mode electronic universal tension tester manufactured by Shanghai Hengyi Precision Instrument Co., Ltd. The test procedure is as follows: a locked sample had two closed loops, one loop, serving as a fixed end, was fixed on a hook of a fixed end of the tension tester, and the other loop penetrated a hook of a moving end of the tension tester, and a moving speed of the moving end of the tension tester was set to 100 µm/min. The maximum value of the sutures being pulled or broken by the tensile testing machine was recorded as the locking force of the locking nail. According to relevant literature, when the locking force is 3N, the clinical requirements for suture tension could be met.

### Fatigue test

The purpose of the fatigue test was to verify whether the locking nail 300 could be used in load as a medical device implant for 10 years after the sutures 500 was locked. Before the test, the two U-shaped folded e-PTFE sutures 500 were locked with the locking nail 300, and then the locking nail 300 and the sutures 500 were placed in a fatigue testing machine that simulates the beating of the left heart system of the human body to perform destructive fatigue testing. The fatigue test equipment adopts an AWT-1000 artificial heart valve fatigue resistance performance tester manufactured by Shanghai Heart Valve Testing Equipment Co., Ltd. The test was performed according to the method of "fatigue test" in ISO 5840 and GB 12279-2008 "Cardiovascular Implant Artificial Heart Valve", where cycle: ≥400 million times; the sliding of the locking nail 300 and damage to the sutures 500 from the locking nails 300, which are caused by load within the fatigue test cycle, were recorded, and whether meeting the relevant requirements of ISO 5840 and GB 12279-2008 "Cardiovascular Implant Artificial Heart Valve" was verified.

The following may be seen from the test results shown in Fig. 16.

When the surface roughness of the first inner cavity section 302 of the locking nail 300 was the same, the deformation rate of the locking nail 300 increased, and the locking force increased accordingly. When the deformation rate of the locking nail 300 was the same, the surface roughness of the first inner cavity section 302 increased, and the locking force increased accordingly.

When the roughness of the first inner cavity section 302 of the locking nail 300 was less than 0.10 µm, the locking force value was too small, and the sutures 500 slipped and caused the fatigue test to fail, which could not meet the fatigue performance requirements.

When the roughness of the first inner cavity section 302 of the locking nail 300 was greater than 2.5 µm, the locking force value was too large, and the sutures 500 were broken, causing fatigue test failure, thereby failing to meet the fatigue performance requirements.

When the deformation rate of the locking pin 300 was less than 0.50, it indicated that the gap was large after the locking pin 300 was deformed, the locking force was too small, the sutures 500 could not be effectively locked, thereby failing to meet the fatigue performance requirements.

When the deformation rate of the locking nail 300 was greater than 0.90, the locking nail 300 was likely to squeeze the sutures 500, and the locking force value of the locking nail 300 tended to decrease, thereby failing to meet the fatigue performance requirements.

Thus, it can be seen that Examples 1-9 in FIG. 16 meet the requirements of better locking ability and fatigue resistance at the same time. In an implementation, the surface roughness of the inner circumference surface of the first inner cavity section 302 of the locking nail 300 of the disclosure ranges from 0.1 µm to 0.25 µm. The deformation rate of the locking portion 310 of the locking nail 300 ranges from 50% to 90%. When the two are matched, the locking portion 310 is deformed to generate the suitable locking force and fatigue resistance to the two U-shaped folded e-PTFE sutures 500 inserted in the locking nail 300. The locking nails in Comparative Examples 1-16 in FIG. 16 do not meet the fatigue performance requirements.

The above is the implementation manners of the implementations of this application. It should be pointed out that those of ordinary skill in the art may also make several improvements and modifications without departing from the principle of the implementations of this application. These improvements and modifications shall fall within the scope of protection of this application.

## Claims

1. A locking nail for locking sutures, comprising a locking portion, wherein the locking nail has a threading cavity used to allow the sutures to extend therethrough, the threading cavity penetrates through the locking portion, the threading cavity has a first inner cavity section located at the locking portion, and a surface roughness of an inner circumference surface of the first inner cavity section ranges from 0.1 µm to 2.5 µm.

2. The locking nail according to claim 1, wherein a deformation rate of the locking portion ranges from 50% to 90%.

3. The locking nail according to claim 1, wherein a deformation rate of the locking portion ranges from 60% to 75%.

4. The locking nail according to claim 1, wherein an inside diameter of the threading cavity ranges from 0.8 µm to 1.2 µm, a thickness of the locking portion ranges from 0.1 µm to 0.2 µm.

5. The locking nail according to claim 1, wherein
the first inner cavity section has a lock boss that is protrusive and a lock groove that is recessed at two opposite sides of the inner circumference surface of the first inner cavity section; and
the locking portion is capable of being deformed when the locking nail is subjected to an external force, so as to allow the lock boss to be pressed into the lock groove.

6. The locking nail according to any of claims 1 to 5, wherein
the locking nail further comprises a retaining portion arranged at a distal end of the locking portion;
the threading cavity further has a second inner cavity section penetrating through the retaining portion, and the second inner cavity section is in communication with the first inner cavity section; and
a surface roughness of an inner circumference surface of the second inner cavity section is less than or equal to that of the inner circumference surface of the first inner cavity section.

7. The locking nail according to claim 6, wherein at least part of the retaining portion is radially protruded from an outer wall of the distal end of the locking portion.

8. The locking nail according to claim 6, wherein both a distal peripheral edge and a proximal peripheral edge of the retaining portion are provided with fillets, the outer surface of the retaining portion is a curved surface.

9. The locking nail according to claim 6, wherein a first smooth transition area is arranged at an intersection of a distal surface of the retaining portion and the inner circumference surface of the first inner cavity section.

10. The locking nail according to claim 9, wherein a surface roughness of the first smooth transition area ranges from 0.1 µm to 2.5 µm.

11. The locking nail according to claim 9, wherein a second smooth transition area is arranged between the first smooth transition area and the inner circumference surface of the first inner cavity section, a surface roughness of the second smooth transmission area is less than 2 µm.

12. The locking nail according to claim 11, wherein the second smooth transition area is a conical surface with an inside diameter gradually decreasing in a direction from a distal end to a proximal end of the threading cavity, a unilateral slope of the conical surface ranges from 0.3 degree to 0.8 degree.

13. The locking nail according to claim 6, wherein an outside diameter of the retaining portion ranges from 1.6 µm to 2.2 µm, and a thickness of the retaining portion ranges from 0.3 µm to 0.6 µm.

14. An interventional remote suture locking device, comprising the locking nail according to any one of claims 1 to 13, further comprising a collet component, wherein the locking nail is inserted in the collet component, the collet component is used for compressing the locking nail to cause the locking portion to deform, so as to lock sutures inserted in the threading cavity of the locking nail.

15. The interventional remote suture locking device according to claim 14, further comprising a driving component and a pushing component arranged at a proximal end of the collet component, wherein the pushing component is used for pushing the collet component and the driving component, and the driving component is used for driving the collet component to compress the locking nail.
